# EUROPEAN PATENT APPLICATION

(11) **EP 3 906 928 A1**
(43) Date of publication of application: **10.11.2021**
(21) Application number: 20173352.4
(22) Date of filing: 06.05.2020
(51) Int. Cl.: A61K 31/505, A61P 31/14

(54) **TREATMENT OF PULMONARY COMPLICATIONS OF CORONAVIRUS**

(71) Applicant: Noorik Biopharmaceuticals AG, 4052 Basel (CH)
(72) Inventor: Navarro, Iker, 4125 Riehen (CH)
(74) Representative: Van den Berg, Frans Richard

(57) **Abstract**

Methods and formulations are provided for the treatment and prevention of the pulmonary complications of coronavirus infections.

## Description

### CROSS-REFERENCE TO RELATED APPLICATIONS

Not Applicable

### FIELD

This invention relates to the treatment and prevention of pulmonary complications resulting from an infection with a coronavirus, in particular hypoxemia, pulmonary edema, pneumonia, Acute Respiratory Distress Syndrome (ARDS) and to related therapeutic and prophylactic formulations.

### BACKGROUND

Viruses of the *coronaviridae* family, commonly referred to as a "coronavirus" or "coronaviruses", are responsible for about 15% of respiratory tract infections, which are mild, self-limiting conditions, sometimes referred to as the "common cold", and in which severe involvement of the lower respiratory tract is infrequent. However, infection with some strains of coronaviruses (for example MERS-CoV, SARS-CoV-1 or SARS-CoV-2) frequently adversely evolve into a severe lower respiratory tract infection with pneumonitis, pneumonia, hypoxemia, Acute Respiratory Distress Syndrome (ARDS) and the need for mechanical ventilation. Pulmonary complications of coronaviruses result in significant morbidity, mortality and disability.

In the winter of 2019, a pandemic caused by the Severe Acute Respiratory Syndrome Coronavirus 2 (or SARS-CoV-2) had resulted in several million people developing Coronavirus Disease 2019 (COVID-19). In only a few months, an unprecedented number of COVID-19 cases had developed, with many displaying severe pulmonary complications, requiring mechanical ventilation and intensive care. Preliminary figures indicate that the mortality of COVID-19 is high. The SARS-CoV-2 virus, colloquially referred to as "coronavirus", is a new, positive-sense, single stranded RNA virus of the *coronaviridae* family and *riboviria* realm, believed to have both animal and human hosts and transmissible between humans. SARS-CoV-2 is related to SARS-CoV-1, the latter also a member of the *coronaviridae* family of viruses and at the center of a global outbreak ("SARS outbreak") between 2002-2004. In the SARS outbreak, 774 fatalities were recorded in about 8,098 confirmed cases (Centers for Disease Control, United States of America - SARS Website, https://www.cdc.gov/sars/about/fs-SARS.pdf, accessed on April 26, 2020). MERS-CoV ("Middle East Respiratory Syndrome Coronavirus"), another member of the *coronaviridae* family, is notable for a limited outbreak originating in the Middle East ("MERS outbreak"), in which a high mortality was observed: 858 fatalities in 2494 confirmed cases, or 34.4% (World Health Organization - MERS Website, https://www.who.int/emergencies/mers-cov/en/, accessed on April 26, 2020). It is not well understood why only some strains of coronaviruses result in pulmonary complications and display a high mortality rate.

In COVID-19, early signs of respiratory insufficiency frequently appear 6 to 7 days after the onset of general symptoms [1]. Patients frequently present with hypoxemia (i.e. low oxygen tension in blood) and difficulty breathing [1], [2]. Some patients with hypoxemia usually progress through a phase of pneumonitis (i.e. lung inflammation), pulmonary edema (i.e. escape of fluid into the alveolar space), pneumonia (i.e. lung inflammation with areas of fluid, protein and cellular exudation into the alveolar space and consolidation) and Acute Respiratory Distress Syndrome (ARDS) within 5 to 7 days, eventually requiring invasive mechanical ventilation and admission to the Intensive Care Unit.

Patients with COVID-19 and early respiratory symptoms have been described to have well ventilated lungs on radiological investigation despite presenting with significant hypoxemia (i.e. low blood oxygen tension) [3]-[6]. This finding is of concern, as the severity of hypoxemia and respiratory symptoms cannot be explained by the radiological appearance of the lung. This has led to the suggestion by some experts that an abnormal regulation of blood flow through the lungs may be a key factor in the impaired gas exchange: adequately ventilated areas of the lung and amenable to gas exchange are inappropriately perfused by blood, and hence result in a condition called ventilation/perfusion mismatch [4]. Some experts have suggested that a state of vasodilation and loss of the ability of the lung to increase vascular resistance in the presence of hypoxemia results in a shunting of blood (i.e. blood that circulates through the lungs, but does not participate in gas exchange), impairing the ability of the blood to exchange carbon dioxide and oxygen. In this respect, some experts have advocated against the use of pulmonary vasodilators as patients with early respiratory problems do not have signs of overt pulmonary hypertension or elevated pulmonary pressures [4], [6].

Endothelin is an endogenous peptide hormone that has two natural receptors, an ETA receptor which mediates vasoconstriction and an ETB receptor which mediates vasodilation, diuresis and clearance of the natural ligand [7], [8]. By modifying vascular resistance and the cross-sectional area of small blood vessels, endothelin participates in the regulation of lung blood flow. Endothelin receptor antagonists, such as ambrisentan, bosentan and macitentan are approved for the treatment of pulmonary hypertension, a condition where pulmonary arterial pressure is increased [7], [9]. Furthermore, the activity of endothelin in the respiratory tract is known to be increased in subjects with upper respiratory airway infections, such as with the respiratory syncytial virus [10]. Experimental data suggest that endothelin may participate in the regulation of the release of inflammatory mediators and the progression of complications in respiratory viral infections [11]-[17]. Experimental blockade of the effects of endothelin reduces the secretion of Vascular Endothelial Growth Factor (VEGF) and Interleukin-6 (IL-6), two cytokines believed to participate in viral-induced lung injury and capillary leakage syndrome [15], [18]. Also experimentally, blockade of the effects of endothelin with bosentan (ETA and ETB receptor blocker) and an ETA-selective anti-sense peptide in chicken infected with the H5N1 strain of influenza (also known as the "avian flu" or "bird flu") have resulted in improved animal survival, in an otherwise universally lethal condition [19], [20].

In this application, endothelin is proposed as key mediator of the vascular pulmonary abnormalities observed in patients with coronavirus infections. As described below, blockade of some of the effects of endothelin in patients with coronaviruses may lead to an improvement of the pulmonary function and result in clinical benefit.

To address the current COVID-19 pandemic, the industrial, regulatory and governmental focus has been to develop potential anti-viral therapies, vaccines and other anti-inflammatory therapies to prevent or treat COVID-19. As vaccines and anti-viral therapies may be effective against one coronavirus in particular, it is in the interest of society to develop therapies that can prevent the common pulmonary complications of all coronaviruses.

### SUMMARY

The inventors have found that the pulmonary complications of a coronavirus infection, in particular hypoxemia, can be prevented and treated by the administration of selective ETA antagonists in amounts that block the endothelin receptor subtype A (ETA) while not significantly blocking the endothelin receptor subtype B (ETB).

The inventors have further found that pulmonary edema associated with a coronavirus infection can be prevented and treated by administering selective ETA antagonists in amounts that block the endothelin receptor subtype A (ETA) while not significantly blocking the endothelin receptor subtype B (ETB).

The inventors have further found that pneumonia associated with a coronavirus infection can be prevented and treated by administering selective ETA antagonists in amounts that block the endothelin receptor subtype A (ETA) while not significantly blocking the endothelin receptor subtype B (ETB).

The inventors have further found that Acute Respiratory Distress Syndrome (ARDS) associated with a coronavirus infection, can be prevented and treated by the administration of selective ETA antagonists in amounts that block the endothelin receptor subtype A (ETA) while not significantly blocking the endothelin receptor subtype B (ETB).

The inventors have further found that selective ETA antagonists interact with and antagonize the ETB receptor when administered at doses approved for the treatment of other conditions such as pulmonary arterial hypertension. In this regard, several endothelin receptor antagonists are approved for oral administration to treat pulmonary arterial hypertension, such as ambrisentan and other compounds discussed below. Some of these, including ambrisentan, are considered ETA selective antagonists. However, their systemic administration in approved dosage forms, notwithstanding their selectivity for the ETA receptor, results in plasma levels that are not ETA receptor selective and therefore are ineffective in preventing and treating pulmonary complications of a coronavirus infection.

In particular, the formulations and methods of the present invention are intended to treat or prevent conditions associated with coronavirus infections.

Achieving the benefits of the present invention requires new and improved formulations for selective ETA antagonists and new methods for their therapeutic use. Accordingly, one objective of the present invention is to provide novel formulations of ETA antagonists and to provide a new medical use of ETA antagonists.

### DESCRIPTION OF INVENTION AND EMBODIMENTS

The present invention relates to a finding that maintaining relatively low blood plasma concentrations of selective ETA antagonists, such as ambrisentan in a preferred embodiment, is effective to treat or prevent the pulmonary complications of a coronavirus infection, in particular hypoxemia. By selectively blocking ETA receptors in pulmonary capillaries, the ETA antagonists maintain capillary patency and reduce the proportion of blood not participating in the gas exchange in the lung. The plasma concentrations of such ETA antagonists are preferably monitored because even selective ETA antagonists may partially antagonize ETB when the ETA are saturated with the antagonist. Because blocking the ETB receptor prevents vasodilation and clearance of endothelin, the clinical benefit provided by the ETA antagonist decreases or even disappears entirely if the effects of endothelin on the ETB receptors are also significantly antagonized. Furthermore, blocking the ETB receptor prevents the normal formation of urine by the kidney, and may represent a life-threatening adverse effect in patients in critical condition.

The present invention and its embodiments are discussed in greater detail below.

### Definitions:

"Acute respiratory distress syndrome" or "ARDS" refers to a type of respiratory failure of rapid onset. Symptoms and signs of ARDS include shortness of breath, tachypnea (rapid breathing), hypoxemia (low blood oxygen arterial tension) and others. ARDS is hereafter referred to a syndrome fulfilling all diagnostic criteria of the Berlin ARDS classification.

"Amount effective to improve hypoxemia", or "amount effective to improve oxygenation" and variations thereof, means that the amount of the administered compound will antagonize or block intra-pulmonary ETA receptors and other cellular components of the lung sufficiently to increase the amount of blood circulating through the lungs and participating in gas exchange, and resulting in an increase in oxygen tension in arterial blood (PaO2). The "amount effective" or "effective amount" refer to an amount effective, at dosages and for periods of time necessary, to achieve the desired therapeutic or prophylactic result. Such effective amounts can be expressed in daily doses of the endothelin receptor antagonist and/or in blood concentrations of the endothelin receptor antagonist, as is discussed herein. For hypoxemia, an effective amount is determined based on the amount necessary to achieve an increase in the oxygen tension in arterial blood (PaO2), an increase in the ratio between the oxygen tension in arterial blood and the inspired fraction of oxygen (PaO2/FiO2 ratio), or a decrease in the proportion of blood circulating through the lung and not participating in oxygen exchange (Qs/Qt or "shunt fraction"). For Pulmonary Edema, an effective amount is determined based on the amount necessary to achieve resolution of signs and symptoms of pulmonary edema (clinical or radiographical), an increase in lung compliance (e.g. decrease in Positive End-Expiratory Pressure required upon mechanical ventilation) or improved oxygenation. For pneumonia, an effective amount is determined based on the amount necessary to achieve resolution of symptoms and radiological signs, an improvement in respiratory function or an increase in the odds of survival. For ARDS, an effective amount is determined based on the amount necessary to achieve an increase in the oxygen tension in arterial blood (PaO2), an increase in the ratio between the oxygen tension in arterial blood and the inspired fraction of oxygen (PaO2/FiO2 ratio), a decrease in the proportion of blood circulating through the lung and not participating in oxygen exchange (Qs/Qt or shunt fraction), a decrease in Positive End-Expiratory Pressure required upon mechanical ventilation, resolution of the radiological signs of ARDS or an increase in the odds of survival.

"Does not significantly antagonize endothelin receptor type B" means that the administered compound does not antagonize ETB at all or to such an extent that the vasodilatory effect of the ETA antagonism is not compromised or adverse renal effects, such as fluid retention and which are secondary to an antagonism of the ETB receptor, are not observed in a patient. When the ETB is significantly antagonized, the increase in pulmonary capillary flow caused by antagonizing ETA is reduced, not observed or even counteracted completely. Such antagonism of ETB can be established by determining the concentration of endothelin in the blood. When ETB is not blocked by the antagonist, the endothelin concentration will be similar to before treatment. When ETB is fully antagonized, the endothelin concentration after treatment with the antagonist will be higher than the level measured before treatment. With preferential ETA blockade, the endothelin concentration in the blood is at most 150% of the endothelin concentration before treatment, more preferably at most 130%, even more preferably at most 120%, and even more preferably at most 110% of the endothelin concentration before treatment. Most preferably, the endothelin concentration is the same in the blood after treatment with the selective ETA antagonist. When ETB is significantly antagonized in a patient, the incidence and severity of adverse renal effects such as weight gain, retention of fluid, formation of ascites, and/or pulmonary edema are increased.

"Improved oxygenation" and variations thereof such as "improvement in arterial oxygen tension", means that a subject who receives the compounds and formulations according to the present invention shows an increase in the total content of oxygen in blood (PaO2), an increase in the ratio between the oxygen tension in arterial blood and the inspired fraction of oxygen (PaO2/FiO2 ratio), or a decrease in the proportion of blood circulating through the lung and not participating in oxygen exchange (Qs/Qt or "shunt fraction"). Such improvement may be associated with beneficial changes, including, for example, decreased incidence and severity of pulmonary edema, a decreased incidence and severity of pneumonia, a decrease in pulmonary arterial hypertension, a decrease in pulmonary vascular resistance or a decreased incidence of ARDS. For example, patients treated according to the present invention may show an increase in arterial blood oxygen tension (PaO2) as determined by arterial gas analysis of at least 10%, preferably at least 20%, an arterial oxygen tension (PaO2) above at least 200 mmHg, preferably at least 300 mmHg, an increase in the ratio between the oxygen tension in arterial blood and the inspired fraction of oxygen (PaO2/FiO2 ratio) of at least 10%, preferably at least 20%, or a decrease in the proportion of blood circulating through the lung and not participating in oxygen exchange (Qs/Qt or shunt fraction) to at most 20%, preferably at most 10% and most preferably at most 5%.

"Individual" or "subject" or "patient" is a mammal. Mammals include, but are not limited to, domesticated animals (for example, cows, sheep, cats, dogs, and horses), primates (for example, humans and non-human primates such as monkeys), rabbits, and rodents (for example, mice and rats). In preferred embodiments, the individual or subject is a human being.

"Coronavirus infection" is an infection of a subject with a virus of the *coronaviridae* family, whether confirmed by testing or by clinical suspicion.

"Liquid composition for parenteral administration" or "liquid formulation for parenteral administration" refers to a preparation which is suitable for intravenous, intraperitoneal, subcutaneous and/or intramuscular administration and in such form as to permit the biological activity of the active ingredient such as an endothelin receptor antagonist, contained therein to be effective, and which contains no additional components which are unacceptably toxic to a subject to which the formulation would be administered. These terms include both compositions that can be directly administered to a subject as well as compositions that need dilution or reconstitution into a conventional parenteral carrier solution. The liquid compositions for intravenous, intraperitoneal, subcutaneous or intramuscular administration may have the same ingredients in the same amounts, but compositions with different ingredients and/or different amounts also are contemplated.

"Liquid composition for oral administration" or "liquid formulation for oral administration" refers to a preparation which is suitable for oral administration and in such form as to permit the biological activity of the active ingredient such as an endothelin receptor antagonist, contained therein to be effective, and which contains no additional components which are unacceptably toxic to a subject to which the formulation would be administered. These terms include both compositions that can be directly administered to a subject as well as compositions that need dilution or reconstitution into a conventional oral carrier solution. Examples of such liquid compositions include oral solutions and oral sprays.

"Pneumonitis" refers to a general inflammation (i.e. the presence of inflammatory cells) of the lung, resulting from a coronavirus infection.

"Pneumonia" refers to the presence in areas of the lung where there is exudation of inflammatory cells, fluid, other cells, debris into the alveolar space, and this exudate is secondary to infection with a coronavirus.

"Pulmonary complications of a coronavirus infection" or "Pulmonary complications associated to a coronavirus infection" refers to the development of adverse changes in or impairment of normal respiratory and/or pulmonary function in a subject suspected to have an infection with a coronavirus. Such complications include hypoxemia, pulmonary edema, pneumonia and ARDS. Pulmonary complications are usually diagnosed by the reporting of symptoms by the subject, the observation of radiological or functional changes in respiratory function or a combination thereof.

"Pulmonary edema" refers to the abnormal accumulation of fluid in the interstitial or alveolar spaces of the lung.

"Shunt Fraction", "Shunting" or "Right-to-Left Shunt" and variations thereof, refers to the hemodynamic phenomenon where a proportion of blood flowing through the lung does not participate in gas exchange. Shunting is a type of ventilation/perfusion mismatch.

"Substantially continuous" means that the administration of the compounds and formulations according to the present invention may be constant or intermittent so long as the indicated blood levels of the ETA inhibitor are maintained, or the intended benefit is obtained.

"Treatment" (and variations thereof such as "treat" or "treating") as well as "prevention" (and variants thereof such as "prevent" or "preventing") refer to clinical intervention in an attempt to alter the natural course of the pathological condition of the individual being treated and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, decreasing the rate of disease progression, amelioration or palliation of the disease state, and remission or improved prognosis.

"Ventilation/Perfusion Mismatch", also referred to as V/Q defects, is a condition in which not all areas of the lung are adequately ventilated (i.e. receive oxygen through the respiratory airway) or perfused (i.e. receive adequate blood flow), resulting in an impaired gas exchange, with the following most common variants: areas of the lung that are ventilated, but not perfused or areas of the lung that are perfused, but not ventilated. It is quantified by the V/Q ratio (volume of air ventilation per minute divided by the cardiac output per minute).

### Treatment of Pulmonary Complications of a Coronavirus Infection

Endothelin plays a central role in the development of pulmonary complications in patients with a coronavirus infection. The activity of endothelin is increased in the lungs by the viral infection and early hypoxemia, resulting in a decrease in the perfusion of pulmonary capillaries by activation of ETA receptors and the inactivation of ETB receptors in the lung, increasing the ventilation/perfusion mismatch and further aggravating hypoxemia. These two effects result in the accumulation of fluid in the alveolar space initially resulting in pulmonary edema. Subsequently, fluid in the alveolar space is infected by other pathogens and there is further exudation of inflammatory cells and debris, resulting in pneumonia. Once a critical volume of the lung is affected, ventilation is significantly affected, resulting in the onset of ARDS. By blocking the effects of endothelin on the ETA receptor, pulmonary capillary flow is increased and inflammation and fluid exudation into the alveolar space are prevented.

Various endothelin receptor antagonists are known, including selective ETA antagonists, such as, for example, sitaxentan, ambrisentan, atrasentan, BQ-123, zibotentan, bosentan, macitentan, tezosentan and darusentan. Preferred endothelin receptor antagonists are sitaxentan, ambrisentan, atrasentan, bosentan and macitentan. More preferred endothelin receptor antagonists are atrasentan, zibotentan and ambrisentan. Even more preferred endothelin receptor antagonists are atrasentan and ambrisentan. The most preferred antagonist is ambrisentan. None of these are approved for the treatment of the pulmonary complications of a coronavirus infection. Instead, some of these are approved for the treatment of pulmonary arterial hypertension (PAH).

Contrary to the methods and formulations of the present invention, the approved dosage and formulations of endothelin receptor antagonists for the treatment of PAH create plasma levels of the endothelin receptor antagonist that significantly antagonize the effects of endothelin on the ETB receptors as well as the effects of endothelin on the ETA receptors in subjects with a coronavirus infection and the pulmonary complications specified above, thereby causing no increase in pulmonary capillary flow or decreased rather than increased pulmonary capillary flow. For example, ambrisentan, sold under the product name of Letairis® in the US and Volibris® in Europe, was approved for daily oral administration at a dosage strength of 5 mg and 10 mg. Plasma levels following one-time administration of Letairis® in patients often reach 700 ng/ml, and generally are found in the range of about 350 ng/ml or 670 ng/ml for the 5 mg and 10 mg doses, respectively. Such concentrations are too high to be effective in treating the pulmonary complications of a coronavirus infection and in fact are counterproductive because they contribute to the decrease in pulmonary capillary flow and hence may aggravate hypoxemia and the ventilation/perfusion mismatch. Furthermore, high concentrations of the endothelin antagonist may lead to the blockade of ETB receptor sites in other organs outside the lung and may induce fluid retention by the kidney. Fluid retention by the kidney is an undesired effect in subjects prone to develop pulmonary edema, ARDS or are under intensive care.

Moreover, subjects with hypoxemia and other pulmonary complications of a coronavirus infection have frequently difficulties to swallow solids, in particular when the upper airway has been intubated and in the elderly. As such, solid formulations of the drugs may be difficult to swallow and may lead to low compliance with the treatment regimen, unlike liquid formulations that are easier to swallow, may be administered parenterally and provide for buccal absorption of the drug.

For all of these reasons, when administered at the approved dosage form and strength, Letairis® (ambrisentan) and other compounds approved to treat PAH are not effective to treat the pulmonary complications of a coronavirus infection. Moreover, use of approved PAH therapies to treat pulmonary complications of a coronavirus infection may be detrimental in restoring an improved oxygenation and may increase the incidence of renal adverse effects.

Accordingly, the methods and formulations of the present invention contemplate the use of ETA antagonists to achieve much lower blood concentrations than are commonly used for approved therapies for PAH. In one embodiment of the present invention oral routes of administration, particularly solutions, sprays, drops, oral dispersible tablets, dispersible oral films, oral films and powders, are suitable for the various indications described in this application. In addition, preferred embodiments of the present invention also involve parenteral routes of administration, particularly intravenous, subcutaneous and transdermal.

In a preferred embodiment, the invention pertains to the use of ambrisentan or a liquid composition comprising ambrisentan in the treatment of pulmonary complications of a coronavirus infection, wherein ambrisentan is administered to a treated subject such that the plasma levels of ambrisentan are maintained below about 20 ng/ml. The invention hence pertains to ambrisentan for use in treating pulmonary complications of a coronavirus infection, wherein ambrisentan is administered to a treated subject such that the plasma levels of ambrisentan are maintained below about 20 ng/ml. Further, the invention provides a method for treating a subject suffering from pulmonary complications of a coronavirus infection wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the prophylactic treatment of pulmonary complications of a coronavirus infection, wherein ambrisentan is administered to a treated subject such that the plasma levels of ambrisentan are maintained below about 20 ng/ml. The invention hence pertains to ambrisentan for use in preventing pulmonary complications of a coronavirus infection, wherein ambrisentan is administered to a treated subject such that the plasma levels of ambrisentan are maintained below about 20 ng/ml. Further, the invention provides a method for treating a subject to prevent pulmonary complications of a coronavirus infection wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of hypoxemia associated with a coronavirus infection, wherein ambrisentan is administered to a treated subject such that the plasma levels of ambrisentan are maintained below about 20 ng/ml. The invention hence pertains to ambrisentan for use in treating hypoxemia associated with a coronavirus infection, wherein ambrisentan is administered to a treated subject such that the plasma levels of ambrisentan are maintained below about 20 ng/ml. Further, the invention provides a method for treating a subject suffering from hypoxemia associated with a coronavirus infection wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of pulmonary edema associated with a coronavirus infection, wherein ambrisentan is administered to a treated subject such that the plasma levels of ambrisentan are maintained below about 20 ng/ml. The invention hence pertains to ambrisentan for use in treating pulmonary edema associated with a coronavirus infection, wherein ambrisentan is administered to a treated subject such that the plasma levels of ambrisentan are maintained below about 20 ng/ml. Further, the invention provides a method for treating a subject suffering from pulmonary edema associated with a coronavirus infection comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of pneumonia associated with a coronavirus infection, wherein ambrisentan is administered to a treated subject such that the plasma levels of ambrisentan are maintained below about 20 ng/ml. The invention hence pertains to ambrisentan for use in treating pneumonia associated with a coronavirus infection, wherein ambrisentan is administered to a treated subject such that the plasma levels of ambrisentan are maintained below about 20 ng/ml. Further, the invention provides a method for treating a subject suffering from pneumonia associated with a coronavirus infection comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of acute respiratory distress syndrome associated with a coronavirus infection, wherein ambrisentan is administered to a treated subject such that the plasma levels of ambrisentan are maintained below about 20 ng/ml. The invention hence pertains to ambrisentan for use in treating acute respiratory distress syndrome associated with a coronavirus infection, wherein ambrisentan is administered to a treated subject such that the plasma levels of ambrisentan are maintained below about 20 ng/ml. Further, the invention provides a method for treating a subject suffering from acute respiratory distress syndrome associated with a coronavirus infection comprising administering ambrisentan wherein an effective amount of ambrisentan is administered to the subject, wherein ambrisentan is administered to the treated subject such that the plasma levels of ambrisentan are maintained below about 20 ng/ml. In a preferred embodiment, the invention pertains to the use of ambrisentan or a liquid composition comprising ambrisentan in the prophylactic treatment of acute respiratory distress syndrome associated with a coronavirus infection, wherein ambrisentan is administered to a treated subject such that the plasma levels of ambrisentan are maintained below about 20 ng/ml. The invention hence pertains to ambrisentan for use in preventing acute respiratory distress syndrome associated with a coronavirus infection, wherein ambrisentan is administered to a treated subject such that the plasma levels of ambrisentan are maintained below about 20 ng/ml. Further, the invention provides a method for treating a subject to prevent acute respiratory distress syndrome associated with a coronavirus infection comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of pulmonary complications of a viral infection, wherein ambrisentan is administered to a treated subject such that the plasma levels of ambrisentan are maintained below about 20 ng/ml. The invention hence pertains to ambrisentan for use in treating pulmonary complications of a viral infection, wherein ambrisentan is administered to a treated subject such that the plasma levels of ambrisentan are maintained below about 20 ng/ml. Further, the invention provides a method for treating a subject suffering from pulmonary complications of a viral infection wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the prophylactic treatment of pulmonary complications of a viral infection, wherein ambrisentan is administered to a treated subject such that the plasma levels of ambrisentan are maintained below about 20 ng/ml. The invention hence pertains to ambrisentan for use in preventing pulmonary complications of a viral infection, wherein ambrisentan is administered to a treated subject such that the plasma levels of ambrisentan are maintained below about 20 ng/ml. Further, the invention provides a method for treating a subject to prevent pulmonary complications of a viral infection wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of hypoxemia associated with a viral infection, wherein ambrisentan is administered to a treated subject such that the plasma levels of ambrisentan are maintained below about 20 ng/ml. The invention hence pertains to ambrisentan for use in treating hypoxemia associated with a viral infection, wherein ambrisentan is administered to a treated subject such that the plasma levels of ambrisentan are maintained below about 20 ng/ml. Further, the invention provides a method for treating a subject suffering from hypoxemia associated with a viral infection wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of pulmonary edema associated with a viral infection, wherein ambrisentan is administered to a treated subject such that the plasma levels of ambrisentan are maintained below about 20 ng/ml. The invention hence pertains to ambrisentan for use in treating pulmonary edema associated with a viral infection, wherein ambrisentan is administered to a treated subject such that the plasma levels of ambrisentan are maintained below about 20 ng/ml. Further, the invention provides a method for treating a subject suffering from pulmonary edema associated with a viral infection comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of pneumonia associated with a viral infection, wherein ambrisentan is administered to a treated subject such that the plasma levels of ambrisentan are maintained below about 20 ng/ml. The invention hence pertains to ambrisentan for use in treating pneumonia associated with a viral infection, wherein ambrisentan is administered to a treated subject such that the plasma levels of ambrisentan are maintained below about 20 ng/ml. Further, the invention provides a method for treating a subject suffering from pneumonia associated with a viral infection comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of acute respiratory distress syndrome associated with a viral infection, wherein ambrisentan is administered to a treated subject such that the plasma levels of ambrisentan are maintained below about 20 ng/ml. The invention hence pertains to ambrisentan for use in treating acute respiratory distress syndrome associated with a viral infection, wherein ambrisentan is administered to a treated subject such that the plasma levels of ambrisentan are maintained below about 20 ng/ml. Further, the invention provides a method for treating a subject suffering from acute respiratory distress syndrome associated with a viral infection comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of hypoxemia, wherein ambrisentan is administered to a treated subject such that the plasma levels of ambrisentan are maintained below about 20 ng/ml. The invention hence pertains to ambrisentan for use in treating hypoxemia, wherein ambrisentan is administered to a treated subject such that the plasma levels of ambrisentan are maintained below about 20 ng/ml. Further, the invention provides a method for treating a subject suffering from hypoxemia wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of pulmonary edema, wherein ambrisentan is administered to a treated subject such that the plasma levels of ambrisentan are maintained below about 20 ng/ml. The invention hence pertains to ambrisentan for use in treating pulmonary edema, wherein ambrisentan is administered to a treated subject such that the plasma levels of ambrisentan are maintained below about 20 ng/ml. Further, the invention provides a method for treating a subject suffering from pulmonary edema comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of pneumonia, wherein ambrisentan is administered to a treated subject such that the plasma levels of ambrisentan are maintained below about 20 ng/ml. The invention hence pertains to ambrisentan for use in treating pneumonia, wherein ambrisentan is administered to a treated subject such that the plasma levels of ambrisentan are maintained below about 20 ng/ml. Further, the invention provides a method for treating a subject suffering from pneumonia comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of pulmonary hypertension, wherein ambrisentan is administered to a treated subject such that the plasma levels of ambrisentan are maintained below about 20 ng/ml. The invention hence pertains to ambrisentan for use in treating pulmonary hypertension, wherein ambrisentan is administered to a treated subject such that the plasma levels of ambrisentan are maintained below about 20 ng/ml. Further, the invention provides a method for treating a subject suffering from pulmonary hypertension comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of hypoxic pulmonary vasoconstriction, wherein ambrisentan is administered to a treated subject such that the plasma levels of ambrisentan are maintained below about 20 ng/ml. The invention hence pertains to ambrisentan for use in treating hypoxic pulmonary vasoconstriction, wherein ambrisentan is administered to a treated subject such that the plasma levels of ambrisentan are maintained below about 20 ng/ml. Further, the invention provides a method for treating a subject suffering from hypoxic pulmonary vasoconstriction comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of high altitude edema, wherein ambrisentan is administered to a treated subject such that the plasma levels of ambrisentan are maintained below about 20 ng/ml. The invention hence pertains to ambrisentan for use in treating high altitude pulmonary edema, wherein ambrisentan is administered to a treated subject such that the plasma levels of ambrisentan are maintained below about 20 ng/ml. Further, the invention provides a method for treating a subject suffering from high altitude pulmonary edema comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the prophylactic treatment of high altitude sickness, wherein ambrisentan is administered to a treated subject such that the plasma levels of ambrisentan are maintained below about 20 ng/ml. The invention hence pertains to ambrisentan for use in prophylactically treating high altitude sickness, wherein ambrisentan is administered to a treated subject such that the plasma levels of ambrisentan are maintained below about 20 ng/ml. Further, the invention provides a method for prophylactically treating a subject suffering from high altitude sickness comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of pulmonary fibrosis, wherein ambrisentan is administered to a treated subject such that the plasma levels of ambrisentan are maintained below about 20 ng/ml. The invention hence pertains to ambrisentan for use in treating pulmonary fibrosis, wherein ambrisentan is administered to a treated subject such that the plasma levels of ambrisentan are maintained below about 20 ng/ml. Further, the invention provides a method for treating a subject suffering from pulmonary fibrosis comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of acute lung injury, wherein ambrisentan is administered to a treated subject such that the plasma levels of ambrisentan are maintained below about 20 ng/ml. The invention hence pertains to ambrisentan for use in treating acute lung injury, wherein ambrisentan is administered to a treated subject such that the plasma levels of ambrisentan are maintained below about 20 ng/ml. Further, the invention provides a method for treating a subject suffering from acute lung injury comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the treatment of acute respiratory distress syndrome, wherein ambrisentan is administered to a treated subject such that the plasma levels of ambrisentan are maintained below about 20 ng/ml. The invention hence pertains to ambrisentan for use in treating acute respiratory distress syndrome, wherein ambrisentan is administered to a treated subject such that the plasma levels of ambrisentan are maintained below about 20 ng/ml. Further, the invention provides a method for treating a subject suffering from acute respiratory distress syndrome comprising administering ambrisentan wherein an effective amount of ambrisentan is administered to the subject, wherein ambrisentan is administered to the treated subject such that the plasma levels of ambrisentan are maintained below about 20 ng/ml.

In a preferred embodiment, the invention pertains to the use of ambrisentan or a liquid composition comprising ambrisentan in the prophylactic treatment of acute respiratory distress syndrome, wherein ambrisentan is administered to a treated subject such that the plasma levels of ambrisentan are maintained below about 20 ng/ml. The invention hence pertains to ambrisentan for use in preventing acute respiratory distress syndrome, wherein ambrisentan is administered to a treated subject such that the plasma levels of ambrisentan are maintained below about 20 ng/ml. Further, the invention provides a method for treating a subject to prevent acute respiratory distress syndrome comprising administering ambrisentan wherein an effective amount of ambrisentan is administered to the subject, wherein ambrisentan is administered to the treated subject such that the plasma levels of ambrisentan are maintained below about 20 ng/ml.

The above embodiments also apply to zibotentan and atrasentan, and their individual embodiments are also included.

### Therapeutic Levels of ETA Antagonist:

In a preferred embodiment of the present invention, the ETA antagonist is ambrisentan. For purposes of the present invention, ambrisentan is administered to a treated subject such that the plasma levels of that compound preferably are maintained below about 20 ng/ml, preferably below about 10 ng/ml, more preferably below about 5 ng/ml, and most preferably below about 2 ng/ml, and generally, the plasma level of ambrisentan is at least 0.001 ng/ml, preferably at least 0.01 ng/ and most preferably at least 0.1 ng/ml. The said plasma levels of ambrisentan refer to the overall ambrisentan concentration in the blood plasma, which include both the ambrisentan bound to proteins present in the blood and the free, unbound ambrisentan present in the blood. The plasma levels or concentration of both bound and unbound ambrisentan can be determined using conventional techniques.

In a further embodiment, ambrisentan is administered to a treated subject such that the plasma levels of unbound ambrisentan (i.e. not bound to protein) are at most 0.1 ng/ml, preferably at most 0.08 ng/ml, more preferably at most 0.06 ng/ml, and most preferably at most 0.05 ng/ml, and generally at least 0.00001 ng/ml, preferably at least 0.0001 ng/ml, and most preferably at least 0.001 ng/ml. The plasma levels or concentration of the free ambrisentan in the blood plasma can be determined by separating the ambrisentan bound to protein and determining the ambrisentan concentration using conventional analytical techniques.

### Administration Protocols:

It is contemplated that ETA antagonists according to the present invention will be administered for a period of time in which such administration provides clinical benefit. In the case the ETA antagonist, in particular ambrisentan, is used to treat the pulmonary complications of a coronavirus infection which is of a temporary nature, the ETA antagonist will be administered for a period of time of up to about twenty-eight days, preferably up to about fourteen days, and most preferably for about seven days. The administration of the ETA antagonist, when administered intravenously, will be substantially continuous.

However, a physician may choose to administer the ETA antagonist in a repeated cycle of, for example, four days, with one or more intervening days in which the ETA antagonist is not administered.

In the case the ETA antagonist, in particular ambrisentan, is used to treat pulmonary complications of a coronavirus infection, specifically hypoxemia, pulmonary edema, pneumonia or acute respiratory distress syndrome, the ETA antagonist will be administered for a prolonged period of time on a daily basis or less often. The prolonged period can be the period up to the patient dies.

In one embodiment of the invention, the frequency of administration of the ETA antagonist, preferably ambrisentan, is more than once a day, more preferably twice a day, and most preferably three times a day. In this way, the concentration of the antagonist in the blood plasma does not exceed 20 ng/mL at any given time, and the actual plasma concentration of the ETA antagonist, preferably ambrisentan, remains sufficiently high to maintain an increase in pulmonary capillary flow.

In one embodiment, ambrisentan is administered to a subject through an oral spray using a spraying device. The oral spray may be an oral solution or a parenteral solution as described below. The invention further pertains to a spraying device comprising an oral spray device wherein the device is capable of spraying the oral solution, in particular the spraying of about 25 to 120 µl of oral solution per spray device actuation. An example of such a spray device is a spray pump. Depending on the subject and the severity of the disease one or more actuations of the spray are necessary per treatment, preferably between 1 and 5 spray actuations. The frequency of administration is as described above. Further, the oral spray can be used for all indications mentioned in this specification.

### Formulations:

Formulations suitable for use in the aforementioned treatments and indications are formulations that upon administration enable the maintenance of the blood plasma level of the ETA antagonist, preferably ambrisentan, below 20 ng/ml. The inventors have found that formulations that are administered orally are suitable, in particular formulations allowing the ETA antagonist, preferably ambrisentan, to be released in the patient's mouth. Examples of such formulations include oral solutions (OS), orally disintegrating films (ODF), orally disintegrating tablets (ODT), oral sprays and powders. Doses that are not administered in solid form have the additional advantage of being appropriate for subjects who have difficulty in swallowing solids, such as subjects suffering from a neurological deficit, neuropsychiatric impairment, endotracheal intubation, mechanical ventilation or older subjects. Oral administration in the form of solution has the advantage that the antagonist is also absorbed in a shorter period of time than alternative administration methods because of absorption in the mouth mucosa, and the desired blood concentration can be reached more readily and more effectively than with solid oral formulations. Additionally, doses administered in the form of oral solutions such as drops or oral sprays can be adjusted in a simple manner, which may be necessary in view of the metabolic and functional characteristics of the subject.

In one embodiment, the formulation is a parenteral formulation. The invention further pertains to a patch comprising the ETA antagonist, preferably ambrisentan.

The invention further pertains to an oral solution. The oral solution of the invention comprises a solvent and an ETA, preferably ambrisentan.

The solvent can be any solvent known in the art that can be suitably used in oral solutions of the invention. Examples of solvents include water, alcohols such as ethanol, glycerin, polyethylene glycol such as PEG300, PEG400 and PEG600; propylene glycol, N-methyl-2-pyrrolidone, and combinations of two or more of these solvents. Of these solvents water, ethanol, propylene glycol and polyethylene glycol or combinations comprising predominantly any one of these solvents are preferred. In another preferred embodiment, the composition of the invention does not contain water as solvent, and more preferably the composition is free from water. In a preferred embodiment, the solvent comprises ethanol and propylene glycol.

In one embodiment of the invention, the oral solution comprises the solvent in an amount of at most 99.999 % by weight (wt%), based on the total weight of the oral solution. Preferably, the solvent is present in an amount of at most 99.995 wt%, more preferably at most 99.99 wt%, and most preferably at most 99.985 wt%, and preferably at least 99 wt%, more preferably at least 99.5 wt%, even more preferably at least 99.8 wt%, even more preferably at least 99.9 wt% and most preferably at least 99.95 wt%, based on the total weight of the oral solution.

In one embodiment of the invention, the oral solution comprises ambrisentan in an amount of at most 1 % by weight (wt%), based on the total weight of the oral solution. Preferably, ambrisentan is present in an amount of at most 0.5 wt%, more preferably at most 0.2 wt%, even more preferably at most 0.1 wt% and most preferably at most 0.05 wt%, and preferably at least 0.0001 wt%, more preferably at least 0.0005 wt%, even more preferably at least 0.001 wt% and most preferably at least 0.0015 wt%, based on the total weight of the oral solution.

The oral solution of the invention may further comprise OS excipients. The OS excipients may be any excipient known in the art that can be suitably used in oral solutions of the invention. Examples of such OS excipients include surfactants such as sodium dodecyl sulfate, sodium lauryl sulfate, polyoxyethylene sorbitan fatty acids (sold under tradename Tween®), sorbitan fatty acid esters (sold under tradename Span®) and polyoxyethylene stearates; rheology modifiers such as cellulose derivatives, alginic acid and polyvinyl pyrrolidone; preservatives such as boric acid, borate salts, sorbic acid, sorbate salts and phenolics; anti-oxidants such as sodium formaldehyde sulphoxylate, butylated hydroxyanisol and butylated hydroxytoluene (BHT); flavors such as mint, licorice and sucralose; sweetening agents such as fructose, mannitol, sorbitol, aspartame and saccharose; saliva stimulating agents such as citric acid, malic acid, tartaric acid, ascorbic acid and lactic acid; and coloring agents such as titanium dioxide, amaranth, sunset yellow and red iron oxide.

In one embodiment of the invention, the oral solution comprises the OS excipient in an amount of at most 15 % by weight (wt%), based on the total weight of the oral solution. Preferably, the OS excipient is present in an amount of at most 10 wt%, more preferably at most 8 wt%, even more preferably at most 7 wt% and most preferably at most 5 wt%, and preferably at least 0.01 wt%, more preferably at least 0.1 wt%, even more preferably at least 0.5 wt% and most preferably at least 1 wt%, based on the total weight of the oral solution.

Typically, the total amount of the endothelin receptor antagonist, the solvent and the OS excipients (when present) add up to 100 wt% in the oral solution of the invention.

The invention further pertains to an oral solution administered with the aid of a spray pump.

The invention further pertains to orally disintegrating films, also referred to as "ODF" or "orodispersible films". The orally disintegrating film of the invention comprises a water-soluble polymer and an ETA, preferably ambrisentan.

The water-soluble polymer can be any polymer known in the art that can be suitably used in ODFs of the invention. Examples of such polymers include natural polymers such as starch, polymerized rosin, pullulan, sodium alginate, pectin, carrageenan, chitosan, gelatin and maltodextrins; and synthetic polymers such as polyvinyl alcohol (PVA), polyethylene oxide (PEO), hydroxypropyl methyl cellulose (HPMC), sodium carboxymethyl cellulose (CMC), polyvinyl pyrrolidone (PVP) and hydroxypropyl cellulose (HPC).

In one embodiment of the invention, the ODF comprises the water-soluble polymer in an amount of at most 99.999 % by weight (wt%), based on the total weight of the ODF. Preferably, the polymer is present in an amount of at most 99.995 wt%, more preferably at most 99.99 wt%, and most preferably at most 99.985 wt%, and preferably at least 99 wt%, more preferably at least 99.5 wt%, even more preferably at least 99.8 wt%, even more preferably at least 99.9 wt% and most preferably at least 99.95 wt%, based on the total weight of the ODF.

In one embodiment of the invention, the ODF comprises ambrisentan in an amount of at most 1 % by weight (wt%), based on the total weight of the ODF. Preferably, ambrisentan is present in an amount of at most 0.5 wt%, more preferably at most 0.2 wt%, even more preferably at most 0.1 wt% and most preferably at most 0.05 wt%, and preferably at least 0.0001 wt%, more preferably at least 0.0005 wt%, even more preferably at least 0.001 wt% and most preferably at least 0.0015 wt%, based on the total weight of the ODF.

The ODF of the invention may further comprise a plasticizer. The plasticizer can be any plasticizer known in the art that can be suitably used in ODFs of the invention. Examples of such plasticizers include polyethylene glycol, glycerol, diethyl phthalate, triethyl citrate and tributyl citrate.

In one embodiment of the invention, the ODF comprises the plasticizer in an amount of at most 15 % by weight (wt%), based on the total weight of the ODF. Preferably, the plasticizer is present in an amount of at most 10 wt%, more preferably at most 8 wt%, even more preferably at most 7 wt% and most preferably at most 5 wt%, and preferably at least 0.01 wt%, more preferably at least 0.1 wt%, even more preferably at least 0.5 wt% and most preferably at least 1 wt%, based on the total weight of the ODF.

The ODF of the invention may further comprise a surfactant. The surfactant can be any surfactant known in the art that can be suitably used in ODFs of the invention. Examples of such surfactants include benzalkonium chloride, sorbitan-based surfactants such as Tween® 20 and Tween 80, block-copolymer of polyethylene glycol and polypropylene glycol such as Poloxamer® 407 and sodium lauryl sulfate.

In one embodiment of the invention, the ODF comprises the surfactant in an amount of at most 15 % by weight (wt%), based on the total weight of the ODF. Preferably, the surfactant is present in an amount of at most 10 wt%, more preferably at most 8 wt%, even more preferably at most 7 wt% and most preferably at most 5 wt%, and preferably at least 0.01 wt%, more preferably at least 0.1 wt%, even more preferably at least 0.5 wt% and most preferably at least 1 wt%, based on the total weight of the ODF.

The ODF of the invention may further comprise ODF excipients. The ODF excipients may be any excipient known in the art that can be suitably used in ODFs of the invention. Examples of such ODF excipients include flavors such as mint, licorice and sucralose; sweetening agents such as fructose, mannitol, sorbitol, aspartame and saccharose; saliva stimulating agents such as citric acid, malic acid, tartaric acid, ascorbic acid and lactic acid; and coloring agents such as titanium dioxide.

In one embodiment of the invention, the ODF comprises the ODF excipient in an amount of at most 15 % by weight (wt%), based on the total weight of the ODF. Preferably, the ODF excipient is present in an amount of at most 10 wt%, more preferably at most 8 wt%, even more preferably at most 7 wt% and most preferably at most 5 wt%, and preferably at least 0.01 wt%, more preferably at least 0.1 wt%, even more preferably at least 0.5 wt% and most preferably at least 1 wt%, based on the total weight of the ODF.

Typically, the total amount of the endothelin receptor antagonist, the water-soluble polymer and additional excipients including the plasticizer, surfactant and ODF excipients (when present) add up to 100 wt% in the ODF of the invention.

The invention further pertains to orally disintegrating tablets, also referred to as "ODT" or "orodispersible tablets". The orally disintegrating tablet of the invention comprises a disintegrant, an ETA, preferably ambrisentan, and optionally a binder.

The disintegrant, also referred to as "superdisintegrant" can be any disintegrant known in the art that can be suitably used in ODTs of the invention. Examples of such disintegrants include crosslinked polyvinyl pyrrolidone such as crospovidone; microcrystalline cellulose; crosslinked cellulose such as Crosscarmellose®, Ac-Di-Sol®, Primellose® and Vivasol®; crosslinked starch such as sodium starch glycolate; sodium carboxymethyl cellulose and hydroxypropyl ethyl cellulose; pregelatinized starch; soya polysaccharides; calcium silicate, and crosslinked alginic acid such as alginic acid NF.

In one embodiment of the invention, the ODT comprises the disintegrant in an amount of at most 99.9 % by weight (wt%), based on the total weight of the ODT. Preferably, the disintegrant is present in an amount of at most 99.5 wt%, more preferably at most 99 wt%, and most preferably at most 98 wt%, and preferably at least 85 wt%, more preferably at least 90 wt%, even more preferably at least 92 wt%, even more preferably at least 93 wt% and most preferably at least 95 wt%, based on the total weight of the ODT.

The binder can be any binder known in the art that can be suitably used in ODTs of the invention. Examples of such binders include polyvinyl pyrrolidone (PVP), polyvinyl alcohol (PVA) and hydroxypropyl methyl cellulose (HPMC).

In one embodiment of the invention, the ODT comprises the binder in an amount of at most 15 % by weight (wt%), based on the total weight of the ODT. Preferably, the binder is present in an amount of at most 10 wt%, more preferably at most 8 wt%, even more preferably at most 7 wt% and most preferably at most 5 wt%, and preferably at least 0.01 wt%, more preferably at least 0.1 wt%, even more preferably at least 0.5 wt% and most preferably at least 1 wt%, based on the total weight of the ODT.

In one embodiment of the invention, the ODT comprises ambrisentan in an amount of at most 1 % by weight (wt%), based on the total weight of the ODT. Preferably, ambrisentan is present in an amount of at most 0.5 wt%, more preferably at most 0.2 wt%, even more preferably at most 0.1 wt% and most preferably at most 0.05 wt%, and preferably at least 0.0001 wt%, more preferably at least 0.0005 wt%, even more preferably at least 0.001 wt% and most preferably at least 0.0015 wt%, based on the total weight of the ODT.

The ODT of the invention may further comprise ODT excipients. The ODT excipients may be any excipient known in the art that can be suitably used in ODTs of the invention. Examples of such ODT excipients include lubricants such as stearic acid, magnesium stearate, zinc stearate, calcium stearate, talc, polyethylene glycol, liquid paraffin, magnesium lauryl sulfate and colloidal silicon dioxide; fillers such as mannitol, sorbitol, xylitol, calcium carbonate, magnesium carbonate, calcium phosphate, calcium sulfate, pregelatinized starch, magnesium trisilicate and aluminium hydroxide; surfactants such as sodium dodecyl sulfate, sodium lauryl sulfate, polyoxyethylene sorbitan fatty acids (sold under tradename Tween®), sorbitan fatty acid esters (sold under tradename Span®) and polyoxyethylene stearates; flavors such as mint, licorice and sucralose; sweetening agents such as fructose, mannitol, sorbitol, aspartame and saccharose; saliva stimulating agents such as citric acid, malic acid, tartaric acid, ascorbic acid and lactic acid; and coloring agents such as titanium dioxide, amaranth, sunset yellow and red iron oxide.

In one embodiment of the invention, the ODT comprises the ODT excipient in an amount of at most 15 % by weight (wt%), based on the total weight of the ODF. Preferably, the ODT excipient is present in an amount of at most 10 wt%, more preferably at most 8 wt%, even more preferably at most 7 wt% and most preferably at most 5 wt%, and preferably at least 0.01 wt%, more preferably at least 0.1 wt%, even more preferably at least 0.5 wt% and most preferably at least 1 wt%, based on the total weight of the ODT.

Typically, the total amount of the endothelin receptor antagonist, the disintegrant, the binder and the ODT excipients (when present) add up to 100 wt% in the ODT of the invention.

In one embodiment, the formulation of the ETA antagonist, preferably ambrisentan, according to the invention is a parenteral formulation. Other forms of parenteral administration also are contemplated, such as subcutaneous, transdermal, intraperitoneal and intramuscular. Of these forms, subcutaneous is preferred. Doses that are not administered orally have the additional advantage of being appropriate for subjects who are unable to swallow, such as subjects suffering from a neurological deficit or hepatic encephalopathy or those subjects under anesthesia or similar conditions. Parenteral administration has the advantage that the antagonist is more effective in a shorter period of time than alternative administration methods, and the desired blood concentration can be reached readily. Additionally, the dose can be adjusted in a simple manner, which may be necessary in view of the metabolic and functional characteristics of the subject.

The present invention further pertains to a liquid composition for parenteral administration comprising an endothelin receptor antagonist, a buffer and a solvent. Preferably, the invention pertains to a liquid composition for parenteral administration comprising ambrisentan, a buffer and a solvent.

The liquid composition of the invention is preferably substantially free of particles. More preferably, the liquid composition of the invention is free of particles. With "particles" is meant any kind of solids including particles of the endothelin receptor antagonist, dust particles or polymeric particles. The term "substantially free of particles" refers to solid particles being present in amounts and sizes as prescribed in the Pharmacopeia and acceptable according to regulatory standards.

In one embodiment of the invention, the liquid compositions comprise the endothelin receptor antagonist, preferably ambrisentan, in an amount of at most 15 % by weight (wt%), based on the total weight of the liquid composition. Preferably, the endothelin receptor antagonist, preferably ambrisentan, is present in an amount of at most 10 wt%, more preferably at most 5 wt%, even more preferably at most 2 wt% and most preferably at most 1 wt%, and preferably at least 0.00001 wt%, more preferably at least 0.0001 wt%, even more preferably at least 0.0005 wt% and most preferably at least 0.001 wt%, based on the total weight of the liquid composition.

In one embodiment of the invention, the liquid composition comprises the buffer in an amount of at most 15 % by weight (wt%), based on the total weight of the liquid composition. Preferably, the buffer is present in an amount of at most 10 wt%, more preferably at most 8 wt%, even more preferably at most 7 wt% and most preferably at most 5 wt%, and preferably at least 0.01 wt%, more preferably at least 0.1 wt%, even more preferably at least 0.5 wt% and most preferably at least 1 wt%, based on the total weight of the liquid composition.

Suitable solvents for use in the compositions of the invention include water, alcohols such as ethanol, glycerin, polyethylene glycol such as PEG300, PEG400 and PEG600; propylene glycol, N-methyl-2-pyrrolidone, and combinations of two or more of these solvents. Of these solvents water, ethanol, propylene glycol and polyethylene glycol or combinations comprising predominantly any one of these solvents are preferred. In another preferred embodiment, the composition of the invention does not contain water as solvent, and more preferably the composition is free from water.

In one embodiment of the invention, the liquid composition comprises the solvent in an amount of at least 85 % by weight (wt%), based on the total weight of the liquid composition. Preferably, the solvent is present in an amount of at least 90 wt% and most preferably at least 92 wt%, and preferably at most 99.9 wt%, more preferably at most 98 wt%, and most preferably at most 95 wt%, based on the total weight of the liquid composition. Typically, the total amount of the endothelin receptor antagonist, the buffer, the solvent and additional excipients (when present) add up to 100 wt% in the liquid compositions of the invention.

When a buffer is present in the liquid composition, the buffer used in the composition of the invention may be any buffer known in the art which can be suitably used for parenteral administration. The buffer generally serves to maintain the composition at a constant pH, in particular upon storage and when the composition is moderately diluted. In one embodiment of the invention, the buffer is chosen such that the pH of the composition is generally between 6 and 12, preferably the pH is at least 6.5, more preferably at least 7, and preferably the pH is at most 10, and more preferably at most 9. Particularly preferred are compositions comprising ambrisentan having a pH of at least 9, as these compositions typically have a better stability and may have a higher concentration of ambrisentan.

In an embodiment of the invention, the pH of the resulting parenteral composition after adding the concentrated liquid composition of the invention is generally between 6 and 12, preferably the pH is at least 6.5, more preferably at least 7, and preferably the pH is at most 9, and more preferably at most 8.

Examples of suitable buffers include ammonium acetate, arginine, sodium benzoate, disodium citrate, trisodium citrate, diethanol amine, hydrobromic acid, monoethanol amine, phosphoric acid, monobasic sodium phosphate, dibasic sodium phosphate, tribasic sodium phosphate, monobasic potassium phosphate, dibasic potassium phosphate, tris(hydroxymethyl)methylamine (Tromethamine or Tris), 4-2-hydroxyethyl-1-piperazineethanesulfonic acid (HEPES), and 2(R)-2-(methylamino)succinic acid, and combinations of two or more of said buffers. It is also contemplated to combine one or more of the mentioned buffers with one or more buffer agents having a pKa below 6 and above 10 as long as the overall pH of the composition of the invention is between 6 and 10.

The liquid composition may be comprised of other components commonly used in liquid compositions for parenteral administration. When the liquid formulation of the invention comprises components other than the endothelin receptor antagonist or ambrisentan, the buffer and the solvent, the total amount of the endothelin receptor antagonist or ambrisentan, the buffer, the solvent, and the other components, such as excipients, add up to 100 wt% of the total weight of the liquid composition.

The compositions of the invention may further comprise excipients. Suitable excipients are known in the art. Such excipients include, stabilizers and/or bulking agents such as mannitol, sucrose, trehalose, polyethylene glycol; tonicity agents like dextrose, sodium chloride, glycerol, glycerin and mannitol; viscosity enhancers or reducers such as sodium carboxymethyl cellulose, acacia, gelatin, methyl cellulose and polyvinyl pyrrolidone; surfactants like polyoxyethylene sorbitan monooleate (Tween 80), sorbitan monooleate, polyoxyethylene sorbitan monolaurate (Tween 20), polyoxyethylene polyoxypropylene copolymers (Pluronics), and lecithin; chelates like calcium disodium ethylenediaminetetra acetic acid (EDTA), disodium EDTA, sodium EDTA, calcium versetamide Na, calteridol and diethylenetriaminepenta acetic acid (DTPA); antioxidants such as acetyl cysteine, sulfurous acid salts (bisulfites and metasulfites), antimicrobial agents like phenol, meta-cresol, benzyl alcohol, methyl paraben, propyl paraben and butyl paraben; and other adjuvants. It is appreciated that some excipients may have multiple properties.

In one embodiment of the invention, the liquid composition comprises the excipient in an amount of at most 15 % by weight (wt%), based on the total weight of the liquid composition. Preferably, the buffer is present in an amount of at most 10 wt%, more preferably at most 8 wt%, even more preferably at most 7 wt% and most preferably at most 5 wt%, and preferably at least 0.01 wt%, more preferably at least 0.1 wt%, even more preferably at least 0.5 wt% and most preferably at least 1 wt%, based on the total weight of the liquid composition.

In a preferred embodiment, the liquid composition of the invention is isotonic. In a preferred embodiment, the liquid composition of the invention is sterile.

The liquid compositions of the invention can be prepared using conventional techniques.

Liquid compositions for parenteral administration can be provided in any suitable container including but not limited to an ampoule, a vial, a pre-filled syringe, a cartridge for a subcutaneous pump, a cartridge for a subcutaneous pen, medication reservoir for a subcutaneous pump or an IV container such as an IV bag or bottle. The concentration of the ETA receptor antagonist may differ depending on the container used in order to achieve clinical benefit.

The liquid compositions may be administered directly to the subject. They also initially may be stored or formulated in concentrated forms that will be diluted in an appropriate parenteral solution, such as conventional physiological solutions for intravenous administration, pharmaceutically acceptable organic solvents such as propylene glycol and ethanol, or combinations thereof, before being administered. Appropriate buffers, excipients and preservatives are conventional. Examples of such parenteral solutions include saline solutions (sterile aqueous solutions of sodium chloride), Ringer's lactate solution, Hartmann's solution (comprising sodium lactate), dextrose-containing solutions (like D5W or D10W) and solutions combining any of the foregoing ingredients (like D5NS or D5LR), and pharmaceutically acceptable organic solvents such as propylene glycol and ethanol, or combinations thereof.

The liquid compositions may be added to the conventional parenteral solutions using conventional techniques. The dilution factor of the endothelin receptor antagonist may be at least 5, which means that, for example, 50 ml of the liquid composition of the invention is added to 200 ml of the conventional parenteral solutions. Preferably, the dilution factor is at least 10 and most preferably at least 15, and generally at most 100, preferably at most 75, and most preferably at most 50.

The invention further pertains to transdermal patches. The transdermal patch may be any known transdermal patch in the art comprising the ETA antagonist, preferably ambrisentan. The transdermal patch of the invention generally comprises the ETA antagonist, preferably ambrisentan, a polymer matrix and/or drug reservoir, optionally a permeation enhancer, optionally a pressure sensitive adhesive, a backing laminate, a release liner and optionally other transdermal patch excipients. Such transdermal patches may be a single-layer-drug-in-adhesive system, a reservoir system, a matrix system such as a drug-in-adhesive system or a matrix-dispersion system, and a micro-reservoir system such as described in Sharma (in Organic & Medicinal Chem IJ 7(2), OMCIJ.MS.ID.555707 (2018), pp.1-5).

The polymer matrix or drug reservoir can be any polymer known in the art that can be suitably used in transdermal patches of the invention. Examples of such polymer matrices include natural polymers such as cellulose derivatives, zein, gelatin, shellac, waxes, gums, natural rubber; synthetic elastomers such as polybutadiene, hydrin rubber, silicon rubber, polyisobutylene, acrylonitrile, neoprene and butyl rubber; and synthetic polymers such as polyvinyl alcohol, polyvinyl chloride, polyethylene, polypropylene, polyacrylate, polyamide, polyuria, polyvinyl pyrrolidone and polymethylmethacrylate.

The permeation enhancer can be any permeation enhancer known in the art that can be suitably used in transdermal patches of the invention. Examples of such permeation enhancers include dimethyl sulphoxide (DMSO), azone, pyrrolidones such as methyl pyrrolidone; fatty acids such as lauric acid, myristyc acid, oleic acid, linoleic acid and capric acid; essential oils such as terpenes and terpenoids; oxazolidinones such as 4-decyloxazolidin-2-one; and urea.

The pressure sensitive adhesive can be any pressure sensitive adhesive known in the art that can be suitably used in transdermal patches of the invention. Examples of such pressure sensitive adhesive include polyacrylates, polyisobutylene and silicon-based adhesives.

The backing laminate can be any backing laminate known in the art that can be suitably used in transdermal patches of the invention. Examples of such backing laminates include vinyl, polyethylene and polyester films.

The release liner can be any release liner known in the art that can be suitably used in transdermal patches of the invention. Examples of such release liners include non-occlusive release liners made of paper fabric, for instance; and occlusive release liners made of, for example, polyethylene and polyvinylchloride.

The transdermal patch of the invention may further comprise TP excipients. The TP excipients may be any excipient known in the art that can be suitably used in transdermal patches of the invention. Examples of such TP excipients include solvents such methanol, chloroform, methanol, acetone, isopropanol and dichloromethane; and plasticizers such as dibutyl phthalate, triethyl citrate, polyethylene glycol and polypropylene glycol.

The size, thickness and amounts of the various features used in the transdermal patch of the invention are conventionally used values.

In general, it is contemplated that persons skilled in the art will adjust the amount of ETA (or ambrisentan) and formulation by conventional means as is appropriate for administration to a particular subject.

The above embodiments of the liquid composition of the invention also apply to zibotentan and atrasentan, and their individual embodiments are also included.

The above embodiments of the orodispersible tablet composition of the invention also apply to zibotentan and atrasentan, and their individual embodiments are also included.

The above embodiments of the orodispersible film composition of the invention also apply to zibotentan and atrasentan, and their individual embodiments are also included.

The above embodiments of the transdermal patch composition of the invention also apply to zibotentan and atrasentan, and their individual embodiments are also included.

### EXAMPLES

### Examples 1 and 2: Preparation of ambrisentan oral solutions

Two oral solutions comprising respectively 500 µg (Example 1) and 1 mg (Example 2) ambrisentan per ml of solvent were prepared. First 5 mL Ethanol and 90 mL propylene glycol 1,2 were mixed together. The resulting solution was heated to 40 °C. Subsequently, the necessary amount of ambrisentan was added and the solution was stirred until the ambrisentan completely dissolved. The resulting solution was cooled and sterilized by passing the solution through a suitable filter. The resulting liquid composition is in accordance with the invention and contains 500 µg/mL of ambrisentan (Example 1) and 1 mg/mL of ambrisentan (Example 2). Analyzing the resulting liquid composition showed that ambrisentan remained stable and no decomposition was observed.

### Example 3: Preparation of Ambrisentan parenteral formulation:

A liquid composition comprising 5 mg ambrisentan per ml of solvent was prepared. First 2750 g Ethanol and 2750 g propylene glycol 1,2 were mixed together. The resulting solution was heated to 40 °C. Subsequently, 30.27 g of ambrisentan were added to 5469.7 g of the solution. The solution was stirred until the ambrisentan completely dissolved. The resulting solution was cooled and sterilized by passing the solution through a suitable filter. The resulting liquid composition is in accordance with the invention and contains 5 mg/mL of ambrisentan. Analyzing the resulting liquid composition showed that ambrisentan remained stable and no decomposition was observed.

The solution obtained can be diluted in various ratios with an aqueous saline and other buffers having pH 6 and above. It is anticipated that prior to human administration, the physician will mix with sterile aqueous solution to obtain the desired ambrisentan concentration.

### Stability tests

The liquid composition of Example 3 was tested for its stability. The tests were conducted by applying 5 ml of the solution in vials which are stored at different temperatures, i.e. 5°C and 25°C for up to 36 months in conditioned chambers. The solutions did not reveal any chemical and physical decomposition of ambrisentan at 5°C and some degradation was observed at 25°C.

### Examples 4-9: Water-free ambrisentan parenteral formulations

Various compositions in accordance with the invention and Example 3 above using a variety of solvents were prepared. To ambrisentan, the solvents or solvent mixtures were added and stirred. An overview of the ingredients of these compositions are presented in the Table 1 below.

**Table 1 - Water-free ambrisentan formulations**

| Example | Solvent | Ambrisentan concentration (mg/ml) |
|---|---|---|
| 4 | Ethanol/propylene glycol 1,2 (3:7) | 1 |
| 5 | Ethanol/propylene glycol 1,2 (3:7) | 10 |
| 6 | Ethanol/polyethyleneglycol 300 (3:7) | 10 |
| 7 | Ethanol | 1 bis 40 |
| 8 | Propylene glycol 1,2 | 1 bis 10 |
| 9 | Polyethyleneglycol 300 | 1 bis 20 |

In addition to the above examples further binary and tertiary solutions can be prepared for this purpose.

The invention described in this specification generally relates to methods and formulations for the treatment and prevention of pulmonary complications of a coronavirus infection comprising the administration of ETA antagonists. While certain exemplary embodiments have been described above in detail and, it is to be understood that such embodiments and examples are merely illustrative of and not restrictive of the broad invention. In particular, it should be recognized that the teachings of the invention apply to variations of the preferred embodiments that are specifically discussed. Modifications, substitutions, changes and equivalents will now occur to those skilled in the art. Thus, it will be understood that the invention is not limited to the particular embodiments or arrangements disclosed, but is rather intended to cover any changes, adaptations or modifications which are within the scope and spirit of the invention as defined by the appended claims.

The references referred to in the description are shown below and form part of the description.
[1] W.-J. Guan et al., "Clinical Characteristics of Coronavirus Disease 2019 in China.," N. Engl. J. Med., pp. 1-13, 2020.
[2] G. Grasselli et al., "Baseline Characteristics and Outcomes of 1591 Patients Infected With SARS-CoV-2 Admitted to ICUs of the Lombardy Region, Italy.," Jama, pp. 1-8, 2020.
[3] F. Zhou et al., "Clinical course and risk factors for mortality of adult inpatients with COVID-19 in Wuhan, China: a retrospective cohort study," Lancet, vol. 395, no. 10229, pp. 1054-1062, 2020.
[4] L. Gattinoni, S. Coppola, M. Cressoni, M. Busana, and D. Chiumello, "Covid-19 Does Not Lead to a 'Typical' Acute Respiratory Distress Syndrome.," Am. J. Respir. Crit. Care Med., pp. 1-5, 2020.
[5] L. Gattinoni et al., "COVID-19 pneumonia : different respiratory treatment for different phenotypes ?," Intensive Care Med., pp. 1-6, 2020.
[6] L. Gattinoni, D. Chiumello, and S. Rossi, "COVID-19 pneumonia: ARDS or not?," Crit. Care, vol. 24, no. 1, p. 154, 2020.
[7] B. Battistini, N. Berthiaume, N. F. Kelland, D. J. Webb, and D. E. Kohan, "Profile of past and current clinical trials involving endothelin receptor antagonists: the novel '-sentan' class of drug.," Exp. Biol. Med. (Maywood)., vol. 231, no. 6, pp. 653-95, Jun. 2006.
[8] M. Yanagisawa et al., "A novel potent vasoconstrictor peptide produced by vascular endothelial cells.," Nature, vol. 332, no. 6163. pp. 411-415, 1988.
[9] M. M. Hoeper, "Definition, classification, and epidemiology of pulmonary arterial hypertension," Semin. Respir. Crit. Care Med., vol. 30, no. 4, pp. 369-375, 2009.
[10] R. Samransamruajkit, K. Moonviriyakit, P. Vanapongtipagorn, N. Prapphal, J. Deerojanawong, and Y. Poovorawan, "Plasma endothelin-1 in infants and young children with acute bronchiolitis and viral pneumonia.," Asian Pacific J. allergy Immunol., vol. 20, no. 4, pp. 229-34, Dec. 2002.
[11] R. Samransamruajkit, S. Gollapudi, C. H. Kim, S. Gupta, and E. Nussbaum, "Modulation of endothelin-1 expression in pulmonary epithelial cell line (A549) after exposure to RSV.," Int. J. Mol. Med., vol. 6, no. 1, pp. 101-105, 2000.
[12] A. C. D'Aprile, L. B. Fernandes, P. J. Rigby, and R. G. Goldie, "Impact of parainfluenza-3 virus infection on endothelin receptor density and function in guinea pig airways," Clin. Sci., vol. 103, no. SUPPL. 48, pp. 345-348, 2002.
[13] M. J. Carr, R. G. Goldie, and P. J. Henry, "Influence of respiratory tract viral infection on endothelin-1-induced potentiation of cholinergic nerve-mediated contraction in mouse trachea," Br. J. Pharmacol., vol. 119, no. 5, pp. 891-898, 1996.
[14] P. J. Henry, M. J. Carr, R. G. Goldie, and A. Y. Jeng, "The role of endothelin in mediating virus-induced changes in endothelin(B) receptor density in mouse airways," Eur. Respir. J., vol. 14, no. 1, pp. 92-97, 1999.
[15] T. C. Carpenter, S. Schomberg, and K. R. Stenmark, "Endothelin-mediated increases in lung VEGF content promote vascular leak in young rats exposed to viral infection and hypoxia," Am. J. Physiol. - Lung Cell. Mol. Physiol., vol. 289, no. 6 33-6, pp. 1075-1082, 2005.
[16] T. C. Carpenter and K. R. Stenmark, "Endothelin receptor blockade decreases lung water in young rats exposed to viral infection and hypoxia," Am. J. Physiol. - Lung Cell. Mol. Physiol., vol. 279, no. 3 23-3, pp. 547-554, 2000.
[17] A. K. Behera, M. Kumar, H. Matsuse, R. F. Lockey, and S. S. Mohapatra, "Respiratory syncytial virus induces the expression of 5-lipoxygenase and endothelin-1 in bronchial epithelial cells," Biochem. Biophys. Res. Commun., vol. 251, no. 3, pp. 704-709, 1998.
[18] M. Browatzki, J. Schmidt, W. Kübler, and R. Kranzhöfer, "Endothelin-1 induces interleukin-6 release via activation of the transcription factor NF-κB in human vascular smooth muscle cells," Basic Res. Cardiol., vol. 95, no. 2, pp. 98-105, 2000.
[19] K. Adachi, R. D. Soejoedono, E. Handharyani, M. Inai, and Y. Tsukamoto, "Therapeutic Trial of an Endothelin Receptor Agonist for the Highly Pathogenic Avian Influenza A/H5N1 Virus Infection in Chicks," Health (Irvine. Calif)., vol. 06, no. 19, pp. 2553-2561, 2014.
[20] M. Imai et al., "Rescue with an anti-inflammatory peptide of chickens infected H5N1 avian flu," Nat. Preced., pp. 4-6, 2009.

## Claims

1. Ambrisentan for use in the treatment of pulmonary complications of a coronavirus infection, wherein ambrisentan is administered to a treated subject such that the plasma levels of ambrisentan are maintained below about 20 ng/ml.

2. Ambrisentan for use in the prevention of pulmonary complications of a coronavirus infection, wherein ambrisentan is administered to a treated subject such that the plasma levels of ambrisentan are maintained below about 20 ng/ml.

3. Ambrisentan for use in the treatment of hypoxemia associated with a coronavirus infection, wherein ambrisentan is administered to a treated subject such that the plasma levels of ambrisentan are maintained below about 20 ng/ml.

4. Ambrisentan for use in the treatment of pulmonary edema associated with a coronavirus infection, wherein ambrisentan is administered to a treated subject such that the plasma levels of ambrisentan are maintained below about 20 ng/ml.

5. Ambrisentan for use in the treatment of pneumonia associated with a coronavirus infection, wherein ambrisentan is administered to a treated subject such that the plasma levels of ambrisentan are maintained below about 20 ng/ml.

6. Ambrisentan for use in the treatment of acute respiratory distress syndrome associated with a coronavirus infection, wherein ambrisentan is administered to a treated subject such that the plasma levels of ambrisentan are maintained below about 20 ng/ml.

7. Ambrisentan for use in the prevention of acute respiratory distress syndrome associated with a coronavirus infection, wherein ambrisentan is administered to a treated subject such that the plasma levels of ambrisentan are maintained below about 20 ng/ml.

8. A method for treating a subject suffering from pulmonary complications of a coronavirus infection comprising administering ambrisentan wherein an effective amount of ambrisentan is administered to the subject, wherein ambrisentan is administered to the treated subject such that the plasma levels of ambrisentan are maintained below about 20 ng/ml.

9. A method for prophylactically treating a subject at risk of developing pulmonary complications of a coronavirus infection comprising administering ambrisentan wherein an effective amount of ambrisentan is administered to the subject, wherein ambrisentan is administered to the treated subject such that the plasma levels of ambrisentan are maintained below about 20 ng/ml.

10. A method for treating a subject suffering from hypoxemia associated with a coronavirus infection comprising administering ambrisentan wherein an effective amount of ambrisentan is administered to the subject, wherein ambrisentan is administered to the treated subject such that the plasma levels of ambrisentan are maintained below about 20 ng/ml.

11. A method for treating a subject suffering from pulmonary edema associated with a coronavirus infection comprising administering ambrisentan wherein an effective amount of ambrisentan is administered to the subject, wherein ambrisentan is administered to the treated subject such that the plasma levels of ambrisentan are maintained below about 20 ng/ml.

12. A method for treating a subject suffering from pneumonia associated with a coronavirus infection comprising administering ambrisentan wherein an effective amount of ambrisentan is administered to the subject, wherein ambrisentan is administered to the treated subject such that the plasma levels of ambrisentan are maintained below about 20 ng/ml.

13. A method for treating a subject suffering from acute respiratory distress syndrome associated with a coronavirus infection comprising administering ambrisentan wherein an effective amount of ambrisentan is administered to the subject, wherein ambrisentan is administered to the treated subject such that the plasma levels of ambrisentan are maintained below about 20 ng/ml.

14. A method for prophylactically treating a subject at risk of developing acute respiratory distress syndrome associated with a coronavirus infection comprising administering ambrisentan wherein an effective amount of ambrisentan is administered to the subject, wherein ambrisentan is administered to the treated subject such that the plasma levels of ambrisentan are maintained below about 20 ng/ml.
